(19) Europäisches Patentamt
European Patent Office
Office européen des brevets

(11) Publication number: 0 323 068
A2

(12) # EUROPEAN PATENT APPLICATION

(21) Application number: 88311745.9

(51) Int. Cl.[4]: C12P 13/00 , C12N 9/88

(22) Date of filing: 12.12.88

(30) Priority: 14.12.87 GB 8729172

(43) Date of publication of application:
05.07.89 Bulletin 89/27

(84) Designated Contracting States:
AT BE CH DE ES FR GB GR IT LI LU NL SE

(71) Applicant: Kent Scientific and Industrial Projects Limited
Physics Laboratory University of Kent
Canterbury Kent CT2 7NJ(GB)

(72) Inventor: Thompson, Lesley Ann
7 Mortimer Street
Hearne Bay Kent, CT6 5PJ(GB)
Inventor: Regan, Andrew Charles
2 Harbledown Terrace Churchill Harbledown
Canterbury Kent, CT2 9AB(GB)
Inventor: Knowles, Christopher John
5 Harbledown Park
Canterbury Kent, CT2 8NR(GB)

(74) Representative: Froud, Clive et al
ELKINGTON AND FIFE Beacon House 113 Kingsway
London WC2B 6PP(GB)

(54) **Enzymatic carbon-carbon bond formation.**

(57) A process for the production of an organic compound by carbon-carbon bond formation between two smaller molecules characterised in that it comprises using a pyridoxal phosphate-linked enzyme having β-cyano-L-alanine synthase and/or cysteine synthase activity to catalyse the reaction without requiring ATP is disclosed.

EP 0 323 068 A2

Xerox Copy Centre

# Enzymatic carbon-carbon bond formation

This invention relates to enzymatic carbon-carbon bond formation; more particularly, it relates to a process for the enzyme-catalysed formation of carbon-carbon bonds in organic molecules without a requirement for ATP (adenosine triphosphate) under mild conditions. By such a biotransformation of two molecules into one larger molecule by carbon-carbon bond formation in accordance with the present invention, stereospecific products may be obtained.

Cyanide is produced as a secondary metabolite by a number of microorganisms and higher plants (see, for example, Knowles, C.J., and Bunch, A.W., Advances in Microbial Physiology, 27, (1986), 73). Cyanide is then detoxified to a number of products. One pathway of detoxification by bacteria and plants is the production of B-cyano-L-alanine. The biotransformation of cyanide consumes L-cysteine and liberates $H_2S$:

L-cysteine + HCN→β-cyano-L-alanine + $H_2S$.

The enzyme β-cyano-L-alanine synthase [E.C.4.4.1.9], which catalyses this biotransformation, is a pyridoxal phosphate-linked enzyme, which acts by β-replacement. β-cyano-L-alanine synthase is known to be produced by cyanogenic plants (see, for example, Hendrickson, H.R., and Conn, E.E. J. Biol. Chem., 244, (1969), 2632).

Cyanide production and its subsequent bioconversion to β-cyano-L-alanine has been extensively studied in Chromobacterium violaceum (see, for example, Macadam, A.M., and Knowles, C.J., Biochimica et Biophysica Acta, 786, (1984), 68). Cyanide is produced from glycine as a secondary metabolite in this bacterium. The conversion to β-cyano-L-alanine occurs during and after the period of cyanide formation (cyanogenesis), by the enzyme β-cyano-L-alanine synthase which is formed in large quantities by the bacterium. The enzyme from C. violaceum has been extensively purified (loc cit). Most interestingly, the β-cyano-L-alanine synthase of C. violaceum is not totally substrate specific. The amino substrate, L-cysteine, may be replaced by either O-acetyl-L-serine or, with very low activity,L-serine:

O-acetyl-L-serine + HCN →β-cyano-L-alanine + acetate
L-serine + HCN→β-cyano-L-alanine + $H_2O$

In addition, it is known that the cyanide co-substrate may be replaced by a number of sulphur-containing compounds producing sulphur-substituted products containing a carbon-sulphur bond. Amongst the sulphur-containing compounds which were able readily to replace the cyanide co-substrate were hydrogen sulphide, ethanethiol, mercaptoethanol, cystamine and dithiothreitol.

It has now surprisingly been found that cyanide or sulphur co-substrates may be replaced by nucleophilic carbon acids or neutral organic compounds. Remarkably, the process according to the present invention results in the formation of a larger organic molecule form two smaller organic molecules due to carbon chain elongation by carbon-carbon bond formation.

The present invention provides a process for the production of an organic compound by carbon-carbon bond formation between two smaller molecules characterised in that it comprises using a pyridoxal phosphate-linked enzyme having β-cyano-L-alanine synthase and/or cysteine synthase activity to catalyse the reaction without requiring ATP.

Generally, the present process is a β-replacement reaction. According to the present invention, a β-cyano-L-alanine synthase obtained from a plant or microorganism source and/or a cysteine synthase obtained from an animal, plant or microorganism source is/are used. Preferably, a β-cyano-L-alanine synthase of C. violaceum and/or a cysteine synthase of E. coli is/are used.

For example, the substrate for the present process may be L-cysteine, L-serine or O-acetyl-L-serine and the co-substrate may be a nucleophilic carbon acid or a neutral carbon species. In one preferred embodiment of the present invention, β-cyano-L-alanine synthase obtained from C. violaceum is used to catalyse the linkage of L-cysteine or O-acetyl-L-serine with nitromethane, malononitrile, methylcyanoacetate or indole. In another preferred embodiment of the present invention, cysteine synthase obtained from E. coliis used to catalyse the linkage of L-cysteine or O-acetyl-L-serine with malononitrile, indole or 5,5-dimethyl cyclohexane-1,3-dione (dimedone).

The present process has no requirement for ATP and, moreover, allows stereospecific synthesis. For example, as the enzyme β-cyano-L-alanine synthase catalyses reactions with L-cysteine or other L-amino acids, but not with the corresponding D-isomers, the resulting products are stereospecific amino acids in the L-configuration. Also, as will be appreciated by those skilled in the art, the stereospecificity generally

inherent in the present invention opens possibilities as regards resolution of racemic mixtures of the amino acid substrates.

As mentioned above, preferred for present purposes is an enzyme exhibiting β-cyano-L-alanine synthase activity obtained from C. violaceum. Another β-replacement, pyridoxal phosphate-linked enzyme, cysteine synthase [E.C.4.2.99.8], the primary activities of which are:

O-acetyl-L-serine + $H_2S$→L-cysteine + acetate

may also exhibit some β-cyano-L-alanine synthase activity (see, for example, Dunnill, P.M., and Fowden, L., Nature, 208, (1965), 1206; Castric, P.A., and Conn, E.E., J. of Bacteriology, 108. (1971), 132; Yanese, H.,et al, Agricultural and Biological Chemistry, 47,(1982), 2925).

L-cysteine + HCN→β-cyano-L-alanine + $H_2S$.

Surprisingly, it has now been shown that, as well as the replacement of the co-substrate ($H_2S$) by alkylthiols (Knowles and Bunch, loc cit), nucleophilic carbon acids or neutral species may also act as alternative co-substrates for cysteine synthase enzymes, resulting in the production of enlarged organic compounds by carbon-carbon bond formation.

The following illustrates the present invention:

Production of C. violaceum cells

Cells of C. violaceum were grown in batch culture under aerobic conditions on a minimal salts medium at 30° C. The medium was supplemented with 20mM glutamate as the sole carbon and nitrogen source. After 20 hours, the cells were harvested by centrifugation, washed twice and resuspended in a minimal volume of Tris buffer (pH 8.5, 50mM).

Production of cell-free enzyme

The harvested and resuspended C. violaceum suspension was sonicated to disrupt the cells and then centrifuged (10,000xg for 10 minutes) to prepare a cell-free extract. In order to prepare a high speed supernatant free of particulate proteins, the cell-free extract was centrifuged at 150,000xg for 5½ hours. The resulting supernatant was decanted and kept as high speed supernatant (HSS).

Purification of HSS

Protamine sulphate was added to HSS to give a final concentration of 1mg per 6mg of protein. The precipitate was removed and the supernatant concentrated by ultrafiltration. The supernatant was then passed down a DEAE-Sephadex A-5O anion exchange column and eluted with a 0-200mM NaCl gradient in Tris buffer (pH 8.5, 300mM). The β-cyano-L-alanine synthase active peak was collected and applied to a Sephadex G-150 gel filtration column. The β-cyano-L-alanine synthase appeared as a single peak. This purification protocol lead to a 99-fold purification of cell-free extract.

Production of E. coli cells

Cells of E. coli $K_{12}$ were grown in batch culture under aerobic conditions on a minimal salts medium at 37° C. The minimal medium was supplemented with 5mM amMonium chloride as a nitrogen source and 20mM glucose as a carbon source. After 24 hours, the cells were harvested by centrifugation, washed twice and resuspended in a minimal volume of Tris buffer (pH 8.5).

Production of cell-free enzyme

The harvested and resuspendedE. coli cells were sonicated to disrupt the cells and then centrifuged

(10,000xg for 10 minutes) to prepare a cell-free extract.

Enzyme assay

Enzyme activity was determined by the rate of $H_2S$ production according to the reaction:

L-cysteine + HCN→β-cyano-L-alanine + $H_2S$

$H_2S$ was detected colourimetrically by the formation of lead sulphide in reaction mixtures containing equal volumes 0.5ml) of cyanide (5mM), L-cysteine (10mM), lead acetate (0.8mM) and enzyme supernatant. The cyanide and L-cysteine were prepared daily in Tris buffer (pH 8.5, 50mM) and the pH adjusted to 8.5. The enzyme's activity was measured by recording the rate of lead sulphide formation per minute. The reaction was also assayed by formation of β-cyano-L-alanine or other products using HPLC (Macadam and Knowles loc cit).

Reactions catalysed by β-cyano-L-alanine synthase of C. violaceum

High speed supernatant containing the β-cyano-L-alanine synthase was examined for activity with L-cysteine and O-acetyl-L-serine and D-cysteine with cyanide as the co-substrate. This work showed that both L-cysteine and O-acetyl-L-serine were suitable amino acid substrates for the enzyme, but D-cysteine could not act as the amino acid substrate.

Experiments proved that the co-substrate cyanide could be replaced by indole. High speed supernatant and purified β-cyano-L-alanine synthase were both used as catalysts and in each case the rate of product formation was between 2 and 9% of the rate with the primary substrate, cyanide:

Primary reaction

L-cysteine + HCN→β-cyano-L-alanine + $H_2S$

Secondary reaction

L-cysteine + indole→L-tryptophan + $H_2S$

Using TLC and HPLC techniques, it was possible to detect tryptophan formation and this showed that tryptophan was produced with $H_2S$ in accordance with the expected stoichiometries of the reaction (i.e. 1:1 tryptophan : $H_2S$). In a reaction mixture with 3mM indole, 95% was converted to tryptophan. Further experiments showed that it was also possible to replace the cyanide co-substrate with other nucleophilic carbon acids or neutral carbon compounds.

For example, the reaction with nitromethane:

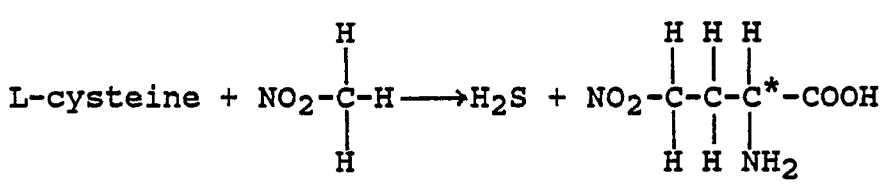

4-nitro-2-amino-butanoic acid

when tested with high speed supernatant, gave a reaction rate of 10-12% of that with the primary substrate.

Malononitrile was a favourable alternative co-substrate:

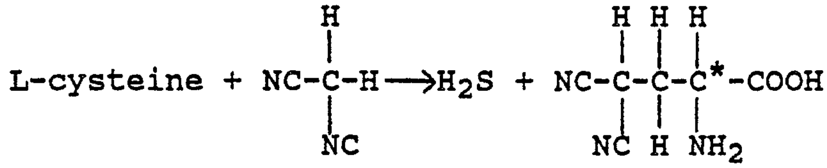

4,4-dicyano-2-amino-butanoic acid

this reaction proceeding at 10-27% of the rate of the primary reaction.

The β-cyano-L-alanine synthase of C. violaceum was also able to catalyse the following reaction of methyl-cyanoacetate at a rate 4-16% of that of the reaction with cyanide:

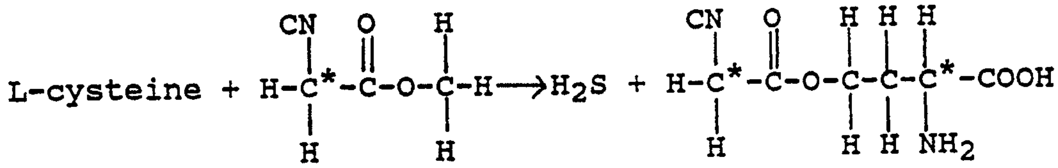

O(cyanoacetyl)serine

Significantly, although the rates for the alternative co-substrates were slower than for the primary reaction, in all cases there were at least 95% yields of the product amino acid formed at the completion of the reactions.

Reactions catalysed by cysteine synthase of E. coli

Supernatant from E. coli $K_{12}$ containing the enzyme cysteine synthase showed activity in the following reaction:

L-cysteine + HCN→β-cyano-L-alanine + $H_2S$

indicating that the cysteine synthase of E. coli does have β-cyano-L-alanine synthase activity. The enzyme was then checked for activity in catalysing the following reactions:

(1) L-cysteine + malononitrile→$H_2S$ + 4,4-dicyano-2-amino-butanoic acid

(2) L-cysteine + indole→$H_2S$ + L-tryptophan

(3) L-cysteine + dimedone→$H_2S$ + 2-(2′-amino-2′-carboxyethyl)-5,5-dimethylcyclohexane-1,3-dione

Reactions (1), (2) and (3) had activities of 2.6, 1.6 and 1.1, respectively, compared to the reaction with L-cysteine and cyanide. D-cysteine was not an amino substrate for any of the reactions.

## Claims

1. A process for the production of an organic compound by carbon-carbon bond formation between two smaller molecules characterised in that it comprises using a pyridoxal phosphate-linked enzyme having β-cyano-L-alanine synthase and/or cysteine synthase activity to catalyse the reaction without requiring ATP.

2. A process as claimed in claim 1 wherein it is a β-replacement reaction.

3. A process as claimed in claim 1 or claim 2 wherein a β-cyano-L-alanine synthase obtained from a plant or microorganism source and/or a cysteine synthase obtained from an animal, plant or microorganism source is/are used.

4. A process as claimed in any of claims 1 to 3 wherein a β-cyano-L-alanine synthase of C. violaceum and/or a cysteine synthase of E. coli is/are used.

5. A process as claimed in any of claims 1 to 4 wherein L-cysteine, L-serine or O-acetyl-L-serine is reacted with a nucleophilic carbon acid or a neutral carbon species.

6. A process as claimed in any of claims 1 to 5 wherein β-cyano-L-alanine synthase obtained from C. violaceum is used to catalyse the linkage of L-cysteine or O-acetyl-L-serine with nitromethane, malononitrile, methyl-cyanoacetate or indole.

7. A process as claimed in any of claims 1 to 5 wherein cysteine synthase obtained from E. coli is used to catalyse the linkage of L-cysteine or O-acetyl-L-serine with malononitrile, indole or dimedone.